# EUROPÄISCHE PATENTANMELDUNG

(11) **EP 2 380 534 A1**
(43) Veröffentlichungstag der Anmeldung: **26.10.2011**
(21) Anmeldenummer: 10160708.3
(22) Anmeldetag: 22.04.2010
(51) Int. Cl.: A61F 7/10

(54) **Kühlvorrichtung**

(71) Anmelder: EMPA Eidgenössische Materialprüfungs- und Forschungsanstalt, 8600 Dübendorf (CH); UNICO swiss tex GmbH, 6053 Alpnachstad (CH)
(72) Erfinder: Weder, Markus, 9428, Walzenhausen (CH); Hess, Markus, 6072, Sachseln (CH)
(74) Vertreter: Felder, Peter

(57) **Zusammenfassung**

Eine Kühlvorrichtung umfasst ein flächiges Kühlelement (E), welches dreilagig ausgebildet ist mit zwei Aussenlagen (2, 4) sowie einem dazwischen angeordneten Verdunstungsbereich (6). Jede Aussenlage ist aus einem wasserdichten und wasserdampfdurchlässigen Material gebildet, und es sind Wasserzuführmittel (8) vorhanden, um den Verdunstungsbereich mit flüssigem Wasser zu versorgen. Angrenzend an die eine Aussenlage (4) ist ein Luft durchströmbares, Abstandhaltendes Ventilationselement (V) angeordnet, welche auf der vom Kühlelement abgewandten Seite zumindest bereichsweise mit einer luftdichten Membrane (M) versehen ist. Zudem ist mindestens ein Aktivlüfter (L) vorhanden, mittels welchem die Ventilationselement von einer Lufteintrittzone (12) zu einer Luftaustrittzone (14) durchlüftbar ist, um aus dem Kühlelement (E) durch die Aussenlage (4) entwichenen Wasserdampf abzuführen.

## Beschreibung

### Technisches Gebiet

Die Erfindung betrifft eine Kühlvorrichtung gemäss Oberbegriff des Anspruchs 1 sowie ein damit ausgestattetes kühlendes Objekt.

### Stand der Technik

In manchen Situationen ist es erforderlich, gewisse Körperpartien oder auch den ganzen Körper einer Person oder eines Tiers vor Überhitzung zu bewahren. Beispiele hierfür sind Personen, die einen Ausdauersport betreiben oder Arbeiter, die in einer heissen Umgebung tätig sind, aber auch Patienten mit gewissen Erkrankungen, deren Symptome durch Kühlung gelindert werden können. Um den jeweils erforderlichen Kühleffekt zu erreichen, sind die verschiedensten Arten von Kühlvorrichtungen entwickelt worden, zu denen insbesondere kühlende Kleidungsstücke oder auch Ausrüstungsteile wie Helme und dergleichen gehören.

In der WO 2005/074846 A1 ist eine Personenkühlvorrichtung beschrieben, die insbesondere für Patienten und ganz besonders für Patienten mit Multipler Sklerose (MS) geeignet ist. Die bekannte Kühlvorrichtung umfasst ein dreilagig ausgebildetes Kühlelement, das gegen die Körperoberfläche einer Trägerperson vorspannbar ist und das je eine Körper zugewandte und eine Körper abgewandte Aussenlage sowie einen dazwischen angeordneten Verdunstungsbereich umfasst. Die Kühlvorrichtung ist zudem mit einem Wasserzuführsystem ausgestattet, um den Verdunstungsbereich mit flüssigem Wasser zu versorgen. Ein wesentlicher Aspekt der bekannten Kühlvorrichtung besteht darin, dass die Aussenlagen aus einem wasserdichten und wasserdampfdurchlässigen Material gebildet sind. Damit wird einerseits verhindert, dass das im Verdunstungsbereich befindliche flüssige Wasser durch die Aussenlagen tritt und so aus dem Kühlelement ausläuft. Andererseits kann Wasserdampf durch die Aussenlagen hindurch entweichen, um so den erwünschten Kühleffekt zu bewirken.

Die bekannte Personenkühlvorrichtung zeichnet sich gegenüber früher vorgeschlagenen Systemen durch einen vergleichsweise einfachen und leichtgewichtigen Aufbau und gleichzeitig durch eine gute Kühlwirkung aus. Beispielsweise wird sie als kühlende Hose für MS-Patienten eingesetzt, wobei die Aussenlagen aus dünnen Polyetheresterschichten mit einer Dicke von 5 bis 10 µm gebildet sind. Mit einer einzelnen Füllung von lediglich 230 Millilitern Wasser pro m² lässt sich die Hautoberfläche während 50 Minuten um bis zu 4°C abkühlen, was bei manchen MS-Patienten zu einer Linderung der Gliederschmerzen und zu einer besseren Beweglichkeit der Extremitäten führt. Dieser Vorgang des Kühlens mit von aussen eingebrachtem Wasser kann beliebig oft wiederholt werden. Da die Aussenlagen des Kühlelements wasserdicht sind, kommt die Haut nicht direkt mit dem flüssigen Kühlwasser in Kontakt und bleibt somit trocken.

Grundsätzlich ist es aus der FR 2 719 882 A bereits bekannt, ein flächiges Kühlelement auf seiner Aussenseite mit einem Ventilationselement zu versehen, um aus dem Kühlelement - im Falle der FR 2 719 882 A durch einen Verdunstungseffekt - eine zusätzliche Kühlung zu erhalten.

Grundsätzlich wäre es denkbar und erwünscht, die bekannte Kühlvorrichtung für eine ganze Reihe von weiteren Anwendungen einzusetzen. Allerdings hat sich herausgestellt, dass die Kühlwirkung in gewissen Situationen nicht ausreicht. Dies ist insbesondere der Fall, wenn die Kühlvorrichtung unter einer vergleichsweise dicken Aussen- oder Schutzbekleidung getragen werden muss, welche nicht oder nur in geringem Masse atmungsaktiv ist, oder wenn sie innerhalb eines Ausrüstungsteils oder eines anderen zu kühlenden Objekts eingebaut ist.

### Darstellung der Erfindung

Aufgabe der Erfindung ist es demnach, eine Kühlvorrichtung der eingangs genannten Art zu erweitern.

Diese Aufgabe wird gelöst durch die im Anspruch 1 definierte Kühlvorrichtung.

Die erfindungsgemässe Kühlvorrichtung umfasst ein flächiges Kühlelement, welches dreilagig ausgebildet ist mit zwei Aussenlagen sowie einem dazwischen angeordneten Verdunstungsbereich, wobei die Aussenlagen je aus einem wasserdichten und wasserdampfdurchlässigen Material gebildet sind und wobei Wasserzuführmittel vorhanden sind, um den Verdunstungsbereich mit flüssigem Wasser zu versorgen. Der Begriff "Lage" für den Verdunstungsbereich ist dabei so zu verstehen, dass zumindest an einigen Stellen ein bestimmter Abstand zwischen den beiden Aussenlagen besteht. Angrenzend an die eine Aussenlage ist ein Luft durchströmbares flächiges Ventilationselement mit ebenfalls einer wasserdampfdurchlässigen Innenschicht und einer zumindest bereichsweise weitgehend luftdichten Membrane angeordnet, zwischen der die Ventilation ausgeführt wird. Durch diesen modularen Aufbau, bei dem die Aussenschicht des Kühlelementes und die dieser zugewandten Innenschicht des Ventilationselementes getrennt voneinander ausgebildet sind, kann offenbar eine verbesserte Konvektion erreicht werden, als wenn - wie bei der FR 2 719 882 A vorgeschlagen, die Wasser führende Schicht des Kühlelementes direkt mit Ventilation beaufschlagt wird oder wenn dazwischen nur eine einfache wasserdampfdurchlässige Trennschicht ausgebildet ist. Weiterhin ist mindestens ein Aktivlüfter vorhanden, mittels welchem die Ventilationsschicht des Ventilationselementes von einer Lufteintrittzone zu einer Luftaustrittzone durchlüftbar ist, um aus dem Kühlelement durch die Aussenlage entwichenen Wasserdampf abzuführen. Es versteht sich, dass die Lufteintrittzone und die Luftaustrittzone derart in einem Abstand zueinander angeordnet sind, dass die durch das flächige Ventilationselement beförderte Luft die zugehörige Aussenlage des Kühlelements möglichst homogen überströmt.

Die erfindungsgemässen Massnahmen haben zur Folge, dass mit der Kühlvorrichtung eine verbesserte Kühlwirkung unter den verschiedensten Anwendungsbedingungen erzielt wird.

Bevorzugte Ausgestaltungen der Erfindung sind in den abhängigen Ansprüchen definiert.

Vorteilhafterweise enthält das Luft durchströmbare flächige Ventilationselement ein Abstandsgewirke, welches eine gute beziehungsweise homogene Durchlüftbarkeit gewährleistet. Hierfür eignet sich beispielsweise ein vernetztes Polyestergarn, das einseitig mit einer luftdichten und optional wasserdampfdurchlässigen Membrane belegt ist. Statt eines Abstandsgewirkes können aber auch andere Abstand haltende Mittel wie Noppen, Stege oder röhrenartige Elemente verwendet werden. Beispielsweise können halbkugelförmige Polyurethan-Noppen mit einem Durchmesser von 2 bis 3 mm verwendet werden. Alternativ können die abstandhaltenden Mittel durch eine Beflockung gebildet sein.

Weiterhin ist es vorteilhaft, wenn das Ventilationselement mit luftdichten seitlichen Abschlüssen versehen ist. Dadurch lässt sich ein unerwünschter seitlicher Abfluss der Ventilationsluft und eine damit einhergehende ungenügende Durchlüftung des gesamten Ventilationselementes vermeiden. Gemäss einer bevorzugten Ausgestaltung ist die Luftaustrittzone durch einen von der luftdichten Membrane freigelassenen Bereich des Ventilationselementes gebildet.

Vorteilhafterweise sind das flächige Kühlelement und die daran angrenzende Ventilationselement zumindest abschnittsweise, vorzugsweise im Randbereich, vorzugsweise mit Klettverschlüssen, Druckknöpfen etc. miteinander verbunden. Dadurch lässt sich eine im Wesentlichen verrutschfreie und einfach anzubringende Kühlvorrichtung realisieren.

Vorteilhaft ist es überdies, wenn der bzw. die Aktivlüfter in das Ventilationselement integriert ist bzw. sind. Mit "integriert" ist hier zu verstehen, dass der Aktivlüfter direkt im Ventilationselement eingebettet ist oder an einen kurzen Schlauchabschnitt angeschlossen ist, der möglichst direkt in das Ventilationselement führt. Wenngleich verschiedene Arten von Aktivlüftern einsetzbar sind, haben sich batteriebetriebene Axial- bzw. Radiallüfter als besonders vorteilhaft erwiesen. Diese zeichnen sich durch eine für die vorgesehene Anwendung sehr kompakte Bauweise sowie durch eine hohe Leistungsdichte aus.

Grundsätzlich kann die Zufuhr des Kühlwassers und die Betätigung des Aktivlüfters manuell geregelt werden. Allerdings kann eine Vorrichtung zur Steuerung des Aktivlüfters vorgesehen sein.

Vorteilhaft ist es überdies, wenn die Dicke der zum Ventilationselement zugewandten Aussenlage geringer ist als die Dicke der dem Ventilationselement abgewandten Aussenlage. Damit wird berücksichtigt, dass die erste Aussenlage einen möglichst geringen Wasserdampf-Durchgangswiderstand aufweisen sollte, um den Austritt des verdunsteten Wassers aus dem Kühlelement zu erleichtern und eine bessere Kühlung zu erreichen. Demgegenüber soll die zweite Aussenlage primär als Barriere gegen den Austritt von flüssigem Wasser wirken; die Durchlässigkeit für Wasserdampf ist dort für die Kühlleistung nicht entscheidend und dient vielmehr dem Abtransport von Feuchtigkeit, die sich angrenzend an die zweite Aussenlage ansammeln kann. Beispielsweise handelt es sich hierbei um Feuchtigkeit, die sich zwischen dem Kühlelement und der Körperoberfläche eines die Kühlvorrichtung tragenden Patienten angesammelt hat. Obwohl mit dieser Kühlvorrichtung die Bildung von Körperschweiss grösstenteils verhindert wird, kann unter gewissen Umständen trotzdem noch Körperfeuchtigkeit in relativ geringen Mengen entstehen, die aber wegen der Wasserdampfdurchlässigkeit des flächigen Kühlelements in das Ventilationselement gelangen kann.

In einer besonders vorteilhaften Ausgestaltung der Erfindung ist das Kühlelement so ausgebildet, dass eine Sogwirkung aus einem Flüssigkeitsreservoir die Zufuhr automatisch durchführt. In einer solchen Ausgestaltung wird der Verdunstungsbereich vorteilhafterweise durch in die zumindest eine der beiden Aussenlagen des Kühlelementes durch eingeprägte kanalartige Strukturen Kapillaren ausgebildet sein. Die flächige Ausgestaltung einer solchen eingeprägten kanalartige Struktur wird vorteilhafterweise als Spiralen oder mäanderförmige Strukturen gebildet. Weiterhin ist es vorteilhaft, wenn die Dicken der kanalartigen Struktur - im Sinne von Blutgefässen - variieren, wobei vorzugsweise die zuführenden Kanäle etwas dicker sind als die abführenden Kanäle. Es sollte in diesem Zusammenhang darauf hingewiesen werden, dass mit einer solchen Ausgestaltung des Kühlelements mit kapillarartigen Kanälen zur Erzielung einer Sogwirkung, insbesondere durch eingeprägte Mikrostrukturen, bereits - im Sinne einer eigenständigen Erfindung - erhebliche und überraschende Effekte auch ohne die Anordnung eines flächigen Ventilationselementes erreicht werden können.

Je nach Anwendungsbereich ist diese Steuerungsvorrichtung mit mindestens einem Temperatursensor oder Temperatur- und Feuchtesensor ausgestattet, um den aktuellen Kühlungsbedarf aufgrund einer Temperatur- resp. Feuchtemessung im zu kühlenden Gebiet zu ermitteln.

Gemäss einer ersten Ausgestaltung handelt es sich hierbei um ein kühlendes Objekt, das von einer Person am oder auf dem Körper getragen wird. Hierzu gehören insbesondere Jacken, Westen, Hosen, Ganzkörperanzüge wie Schutzanzüge und Kühlanzüge, sowie Schutzhelme. Es versteht sich, dass die integrierte Kühlvorrichtung so angeordnet ist, dass sich das Ventilationselement auf der dem Körper abgewandten Seite des kühlenden Objekts befindet. Die vom Ventilationselement abgewandte Aussenlage wird entweder direkt an der Körperoberfläche getragen oder sie ist von der Körperoberfläche lediglich durch eine dünne Lage von Unterwäsche oder dergleichen getrennt. Grundsätzlich kann die luftdichte Membrane des Ventilationselements als Aussenlage des kühlenden Objekts, insbesondere einer kühlenden Jacke, Weste, Hose oder eines Ganzkörperanzugs wirken. Alternativ befindet sich über der luftdichten Membrane des Ventilationselements noch mindestens eine weitere Lage des kühlenden Objekts.

Bei den weiteren Ausgestaltungen ist die integrierte Kühlvorrichtung so angeordnet, dass sich das Ventilationselement auf derjenigen Seite des kühlenden Objekts befindet, die nicht für den Kontakt mit dem zu kühlenden Objekt oder Körperteil vorgesehen ist.

Je nach Anwendungsbereich kann die Kühlvorrichtung bzw. das kühlende Objekt mit Solarzellen ausgestattet werden, die zumindest einen Teil der elektrischen Energie zum Betrieb eines oder mehrerer Aktivlüfter liefern.

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können.

### Kurze Beschreibung der Zeichnungen

Ausführungsbeispiele der Erfindung werden nachfolgend anhand der Zeichnungen näher beschrieben, dabei zeigen:
- Fig. 1: eine Kühlvorrichtung gemäss einer ersten Ausführung der vorliegenden Erfindung, in Draufsicht;
- Fig. 2: die Kühlvorrichtung der Fig. 1, in Schnittdarstellung gemäss dem Schnitt II-II;
- Fig. 3: einen Ausschnitt der Kühlvorrichtung der Fig. 2;
- Fig. 4: einen kühlenden Sessel, in vertikaler Schnittdarstellung.
- Fig. 5: einen kühlenden Rucksack, in vertikaler Schnittdarstellung.
- Fig. 6: eine Kühlvorrichtung gemäss einer zweiten Ausführung der vorliegenden Erfindung, in Draufsicht; und
- Fig. 7: eine erste Variation der flächigen Anordnung der Kapillaren gemäss des zweiten Ausführungsbeispiels nach Figur 6;
- Fig. 8: eine zweite Variation der flächigen Anordnung der Kapillaren gemäss des zweiten Ausführungsbeispiels nach Figur 6;
- Fig. 9: eine erste Variation der geprägten Mikrostruktur der Kapillaren gemäss des zweiten Ausführungsbeispiels nach Figur 6 bzw. 7 und 8;
- Fig. 10: eine zweite Variation der geprägten Mikrostruktur der Kapillaren gemäss des zweiten Ausführungsbeispiels nach Figur 6 bzw. 7 und 8;
- Fig. 11: eine dritte Variation der geprägten Mikrostruktur der Kapillaren gemäss des zweiten Ausführungsbeispiels nach Figur 6 bzw. 7 und 8;

### Wege zur Ausführung der Erfindung

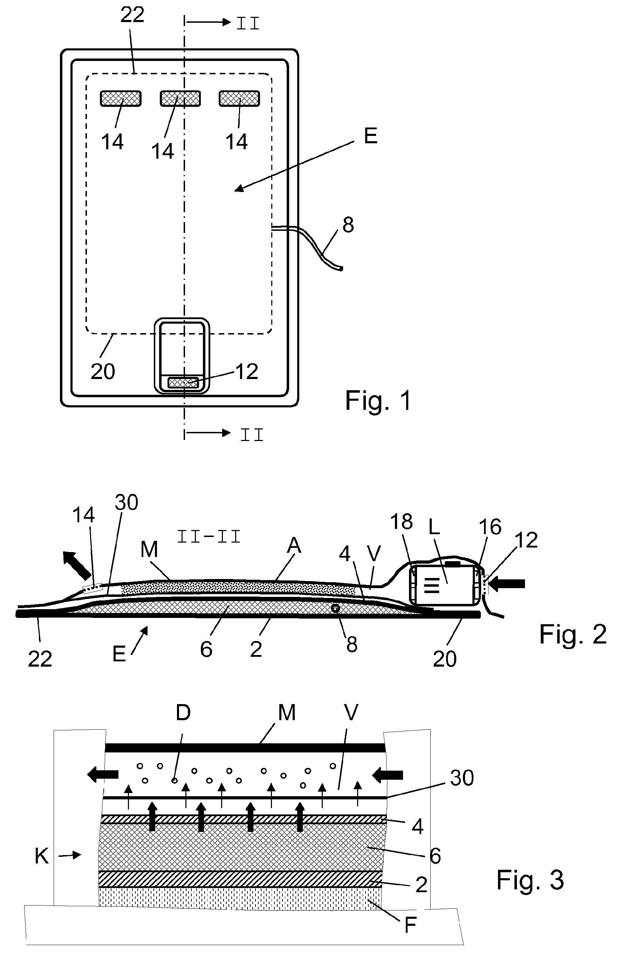

Die in der Figur 1 dargestellte Kühlvorrichtung umfasst - wie in den Figuren 2 und 3 im Einzelnen dargestellt - ein dreilagig ausgebildetes, flächiges Kühlelement E mit zwei Aussenlagen 2, 4 sowie einem dazwischen angeordneten Verdunstungsbereich 6. Beide Aussenlagen 2, 4 sind aus einem wasserdichten und wasserdampfdurchlässigen Material gebildet. Hierfür eignen sich beispielsweise dünne (5 bis 10 µm) Membranen aus Polyetherester (z.B. Sympatex™) oder aus Polyurethan. Insbesondere sollten die Schichten einen vergleichsweise niedrigen Wasserdampf-Durchgangswiderstand Ret aufweisen; so sollte der nach ISO 11092 gemessene Ret-Wert maximal 2 bis 4 m²Pa/W betragen.

Der als Hohlraum zwischen den beiden Aussenlagen 2, 4 ausgebildete Verdunstungsbereich 6 ist im gezeigten Beispiel mit einem hydrophilen Material ausgefüllt, das einerseits als abstandhaltendes Element zwischen den beiden Aussenlagen und andererseits als Wasserspeicher wirkt. Beispielsweise handelt es sich dabei um eine hydrophile Polyesterlage mit einer Schichtdicke von ca. 0.5 mm.

Die Kühlvorrichtung umfasst im hier beschriebenen Ausführungsbeispiel in den Figuren nicht näher dargestellte Wasserzuführmittel, um den Verdunstungsbereich 6 über mindestens einen Zuführschlauch 8 mit flüssigem Wasser zu versorgen.

Weiterhin weist die in den Figuren 1 bis 3 dargestellte Kühlvorrichtung ein sich auf einer Seite des Kühlelementes, anschliessend an den Verdunstungsbereich 6, angeordnetes Ventilationselement V auf. Das Ventilationselement V umfasst eine weitere, im wesentlichen wasserdichte, in jedem Fall aber wasserdampfdurchlässige Membrane 30, eine nach aussen hin abschliessende Membrane M sowie eine dazwischen liegende Ventilationsschicht.

Als Membrane M kann beispielsweise eine Schicht aus Polyetherester oder Polyurethan verwendet werden. Bei gewissen Anwendungen ist es erforderlich, dass die Membrane M nicht nur für Luft, sondern auch für Wasserdampf undurchlässig ist. Für andere Anwendungen kann es jedoch zweckmässig sein, dass die Membrane M für Wasserdampf durchlässig ist, damit die Feuchtigkeit schneller wegtransportiert wird und nicht alle Feuchte über die ventilierte Luft weggeblasen resp. weggesaugt werden muss.

Das Kühlelement ist - wie in Figur 3 dargestellt - angrenzend an eine zu kühlende Fläche F angeordnet, welche beispielsweise ein Hautareal einer Person sein kann. Im gezeigten Beispiel liegt die erste Aussenlage 2 des Kühlelements E direkt an der zu kühlenden Fläche F an. Es ist aber auch möglich, zwischen der ersten Aussenlage 2 und der zu kühlenden Fläche F eine dünne Zwischenschicht, beispielsweise eine Unterwäscheschicht zu haben. Grundsätzlich kann der im Verdunstungsbereich 6 entstehende Wasserdampf sowohl durch die erste Aussenlage 2 wie auch durch die zweite Aussenlage 4 hindurch treten. Der durch die zweite Aussenlage 4 in das Ventilationselement V diffundierende Wasserdampf - in der Fig. 3 durch kleine Kreise D symbolisiert - wird allerdings sogleich durch den Luftstrom (waagrechte Pfeile in der Fig. 3) wegbefördert, sodass weiterer Wasserdampf aus dem Kühlelement E ins Ventilationselement V diffundieren kann. Auf Grund des Partialdrucks, welcher nach aussen wirkt, tritt das im Inneren des Kühlelements E verdunstende Wasser praktisch ausschliesslich durch die zweite Aussenlage 4 und die wasserdichte, wasserdampfdurchlässige Membrane 30 hindurch und wird im Ventilationselement abtransportiert. Die luftdichte Membrane M auf der vom Kühlelement E abgewandten Seite kanalisiert den erforderlichen Luftstrom wie gewünscht. Die Ventilationsschicht des Ventilationselementes V enthält ein - in Figur 2 dargestelltes - Abstandsgewirke A.

Dabei hat sich - etwas überraschend - herausgestellt, dass das sich zwischen dem Kühlelement E und dem Ventilationselement V teilweise - insbesondere, wenn sich ein kleiner Abstand dazwischen ausbilden kann - ein Mikroklima bilden kann, welches die Diffusion begünstigt.

Dieser Effekt kann noch verstärkt werden, wenn die zweite Aussenlage 4 dünner als die erste Aussenlage 2 ist. Es versteht sich, dass durch die Verdunstung des im Verdunstungsbereich 6 befindlichen Wassers ein Kühleffekt eintritt, der insbesondere auf die zu kühlende Fläche F wirkt.

Schliesslich ist mindestens ein Aktivlüfter L vorhanden, mittels welchem innerhalb des Ventilationselementes V von einer Lufteintrittzone 12 zu einer Luftaustrittzone 14 durchlüftbar ist. Hierfür kann beispielsweise ein kompakter batteriebetriebener Axiallüfter mit einer Höhe von ca. 5 mm und einem Durchmesser von ca. 25 mm eingesetzt werden.

Im gezeigten Beispiel wird die Lufteintrittzone 12 durch eine nahe bei der Ansaugseite 16 des Aktivlüfters L ausgebildete Öffnung in der Membrane M gebildet. Die Ausblasseite 18 des Aktivlüfters L befindet sich nahe bei einer ersten Randzone 20 des Kühlelements K. Die Luftaustrittzone 14 wird durch drei weitere Öffnungen in der Membrane M gebildet, welche sich nahe bei einer zweiten Randzone 22 des Kühlelements E befinden, die von der ersten Randzone 20 abgewandt ist. Durch diese Anordnung wird bewirkt, dass das an das Kühlelement E angrenzende Ventilationselement V im Wesentlichen vollumfänglich durch die vom Aktivlüfter L beförderte Luft durchströmt wird. Nebst der beschriebenen Lufteinblasmethode ist es in gewissen Anwendungen vorteilhaft die Luft mittels eines Lüfters an der Austrittsöffnung 14 wegzusaugen und am Lufteinlass 12 frei einströmen zu lassen.

Die Verbindung der beiden Elemente E und V ist im Ausführungsbeispiel mit Klettverschluss, alternativ mit Druckknöpfen oder mit einem Reissverschluss vorgesehen. Dadurch wird das bereits zuvor beschriebene Mikroklima, welches sich teilweise zwischen dem Kühlelement - nämlich seiner Schicht 6 - und dem Ventilationselement - nämlich seiner Schicht 30 - ausbildet, gefördert, indem ein kleiner Abstand zwischen diesen beiden Schichten nicht ausgeschlossen wird.

Zu dem hier beschriebenen ersten Ausführungsbeispiel können noch folgende Details angegeben werden.

Ein typisches Kühlelement gemäss dem hier beschriebenen Ausführungsbeispiel weist 15 um dicke, hydrophile PES-Membranen auf beiden Seiten und eine hydrophile mittlere Feuchteverteilschicht mit einer Dicke ca. 0.4 mm auf.

Eine typisches Ventilationselement V weist ein Abstandsgewirke mit einer Dicke von 5 mm mit Längskanälen, beidseitig mit einer PU-Membrane mit einer Dicke von 20 um laminiert. Die Aufbringung der Membrane auf das Abstandsgewirke geschieht mittels Wärme und Druck (Hotmelt-Klebepunkte). Die Lüfter können typischerweise Lüfter mit eine Leistungsaufnahme von 0.5 W sein.

Wie nachfolgend an ausgewählten Beispielen erläutert, können derartige Kühlvorrichtungen in den verschiedensten Anwendungsbereichen für die Realisierung von kühlenden Objekten eingesetzt werden. Je nach Anwendung ist es von Vorteil, die Kühlvorrichtung K insgesamt eher steif oder eher biegsam auszugestalten. Eine vergleichsweise steife Kühlvorrichtung K lässt sich insbesondere durch entsprechende Wahl des Materials der luftdichten Membrane M und optional durch den Einbau versteifender Elemente in der Ventilationselement V realisieren. Derartige Versteifungselemente können gleichzeitig als Luft leitende Elemente zur Kanalisierung des Luftstroms durch die Ventilationselement V verwendet werden. Es versteht sich, dass eine vergleichsweise steife Kühlvorrichtung V nicht zwangsläufig planar sein muss, sondern beispielsweise konvex bzw. konkav geformt sein kann.

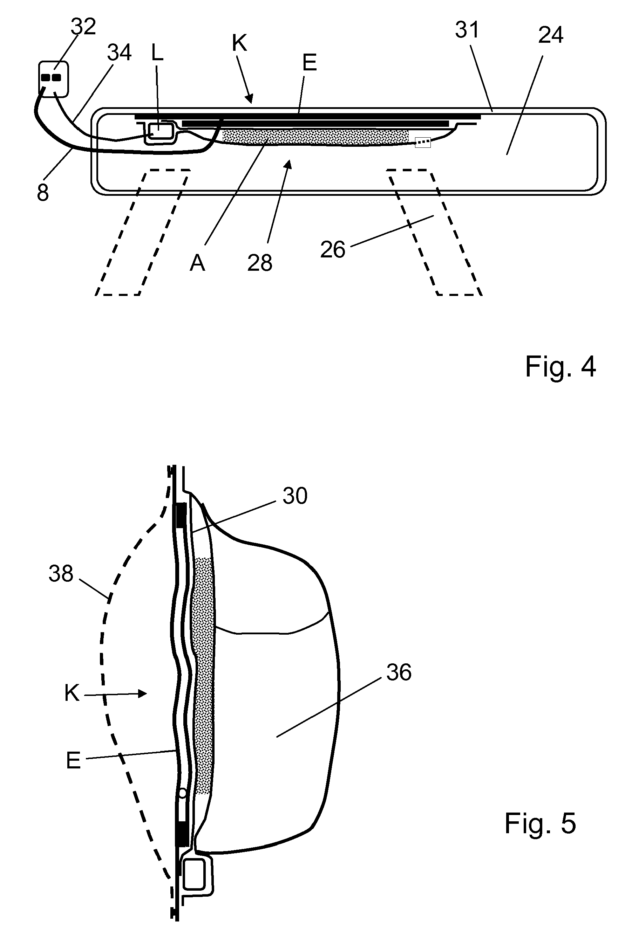

Die Fig. 4 zeigt einen kühlenden Sessel mit einem Sitzelement 24 und schematisch angedeuteten Beinen 26. Das Sitzelement 24 weist an seiner Oberseite eine Ausnehmung 28 auf, in welcher eine Kühlvorrichtung K der oben beschriebenen Art eingelegt ist, wobei das Ganze vorteilhafterweise durch einen abnehmbaren Überzug 31 abgedeckt ist. Beispielsweise ist der Überzug 31 mit Klettverschlüssen oder Knopfverbindungen ausgestattet. Wie aus der Fig. 5 hervorgeht, ist die Kühlvorrichtung K in der Ausnehmung 28 derart angeordnet, dass das Kühlelement E nach oben weist und somit direkt unter dem Überzug 31 liegend einen Teil der Sitzfläche des kühlenden Sessels bildet.

Es ist bekannt, dass bei langem Sitzen bei wärmeren Umgebungsbedingungen ein Temperaturanstieg in den männlichen Hoden stattfinden kann und dementsprechend sich die Spermienqualität deutliche verschlechtert. Mit der oben beschriebenen Einrichtung kann z.B. ein Spezialstuhl hergestellt werden, bei welchem gezielt der Bereich um die männlichen Hoden gekühlt wird.

Die Kühlvorrichtung K ist mit einem Strom- und Wasserversorgungsmodul 32 ausgestattet, das über ein Stromversorgungskabel 34 mit dem Aktivlüfter und über einen Wasserzuführschlauch 8 mit dem Kühlelement E verbunden ist. Im gezeigten Beispiel weist das Modul 32 ein Gehäuse auf, in welchem eine oder mehrere Batterien oder Akkus sowie ein befüllbarer Wasserbehälter und eine Dosierpumpe untergebracht sind. Vorteilhafterweise umfasst das Modul 32 eine programmierbare Steuereinheit auf, mit welcher der Betrieb des Aktivlüfters sowie die Wasserversorgung des Kühlelements in aufeinander abgestimmter Weise gesteuert werden. Optional ist zudem ein Sensor zur Überwachung des Füllstands im Kühlelement E vorhanden. Je nach Anwendung und Ausgestaltung des Sessels kann das Modul 32 beispielsweise an einer Armlehne oder an einem Halteelement am Seitenrand des Sitzelements 24 angebracht werden. Im gezeigten Beispiel dient die Kühlvorrichtung K dazu, die Sitzfläche des damit ausgerüsteten Sessels nach individuellem Bedarf einer darauf sitzenden Person zu kühlen. Durch Verwendung eines Temperatur und/oder Drucksensors ist es möglich, die Kühlung erst dann zu aktivieren, wenn jemand auf dem Sessel sitzt. Mittels eines Temperatursensors in der Steuerungseinheit 32 kann eine von der Umgebungstemperatur abhängige Kühlwirkung eingerichtet werden, bei der automatisch die Lüftung und die Wasserzufuhr der erforderlichen Kühlwirkung angepasst werden.

Während die Fig. 4 beispielhaft einen kühlenden Sessel zeigt, können auf ganz analoge Art und Weise auch Stühle, Hocker, Sofas, aber auch Schlafdecken, Kissen, Matratzen und Luftmatratzen mit einer oder mehreren Kühlvorrichtungen E ausgestattet werden. Wesentlich ist dabei, dass die Steifigkeit der abstandhaltenden Lage, beispielsweise also des Abstandsgewirkes A, genügend hoch ist, um ein Zusammenfallen des Ventilationselements V zu verhindern. Für die Anwendung in einem Sessel oder dergleichen muss das Abstandsgewirke A das Gewicht einer darauf sitzenden Person aushalten können und muss demnach vergleichsweise steif sein. Für die Anwendung in einem Kissen oder einer Decke wird in aller Regel eine geringere Steifigkeit erforderlich sein.

Die Fig. 5 zeigt einen kühlenden Rucksack mit einer beutelartigen Kammer 36 und schematisch dargestellten Trageriemen 38. Auf derjenigen Seite der Kammer 36, welche der den Rucksack tragenden Person zugewandt ist, befindet sich eine Kühlvorrichtung K der oben beschriebenen Art. Dabei ist die Kühlvorrichtung K so ausgerichtet, dass sich deren Kühlelement E auf der den Rucksack tragenden Person zugewandten Seite befindet. Vorteilhafterweise ist der kühlende Rucksack wie im obigen Beispiel mit einem Strom- und Wasserversorgungsmodul 32 ausgestattet, dass je nach Bedarf an einem der Trageriemen 38 oder an der Aussenseite bzw. in einer Seitentasche der Rucksack-Kammer 36 befestigt bzw. untergebracht werden kann.

In weiteren, hier nicht näher dargestellten Anwendungen können eine oder mehrere Kühlvorrichtungen K der oben beschriebenen Art für den Einsatz in anderen kühlenden Objekten wie beispielsweise Jacken, Westen, Hosen, Ganzkörperanzügen und Schutzhelmen verwendet werden. Insbesondere beim Einsatz in Helmen ist es von Vorteil, wenn die Kühlvorrichtung K eine entsprechend konkave Formgebung aufweist. Beim Einsatz in Ganzkörperanzügen werden in aller Regel mehrere Kühlvorrichtungen K für verschiedene Partien des Anzugs benötigt. Dabei wird es zweckmässig sein, ein Strom- und Wasserversorgungsmodul mit Steuerung zu verwenden, das sämtliche Kühlvorrichtungen unabhängig und allenfalls gleichzeitig bedienen kann.

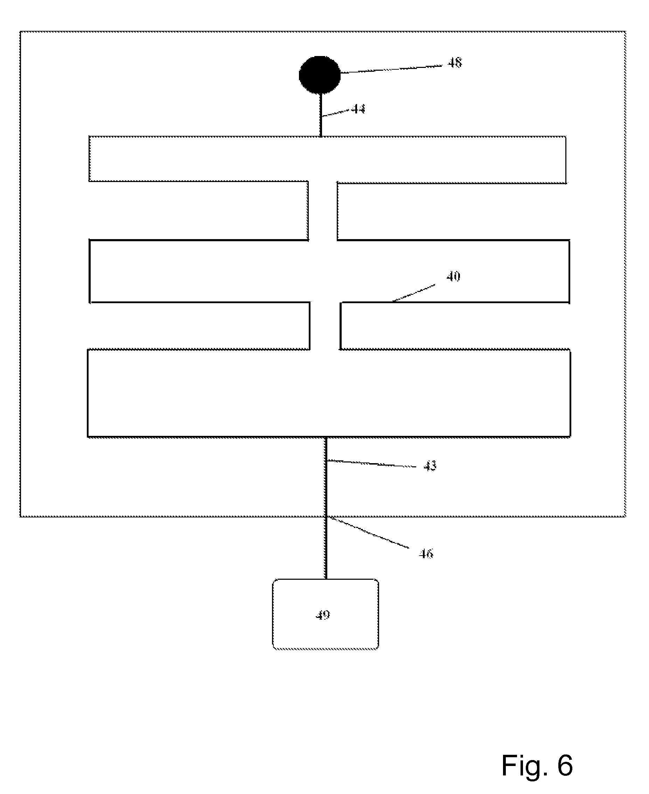

In den Figuren 6 bis 11 sind verschiedene Variationen zu weiteren, alternativen Ausführungsbeispielen dargestellt, bei dem das Kühlelement E jeweils in einer besonderen Art optimiert ist. Dabei sind die Membranen so strukturiert, dass - wenn beispielsweise 2 Membranen miteinander verklebt werden - der Verdunstungsbereich 6 durch Kapillare gebildet durch die mikrostrukturierten Oberflächen der Membrane ausgebildet sind und insofern keine spezielle Feuchteverteilschicht notwendig ist. Die kapillaren Saugeffekte der mikrostrukturierten Oberfläche übernehmen dann die Funktion der mittleren hydrophilen Feuchteverteilschicht. Es wurde nämlich festgestellt, dass dank der beiden Membranen 2, 4 ein Unterdruck erzeugt wird der für viele Anwendungen sogar eine zusätzliche Wasserpumpe überflüssig macht. Dieser sogenannte Transpirationssog entsteht, wenn Wasserdampf verdunstet und so einen Unterdruck in der Kühlelement erzeugt, analog den Blättern eines Baums. Eine Kapillare mit einem Radius von 0.1 mm ergäbe beispielsweise eine Saughöhe von 14 cm.

In diesen alternativen Ausführungsbeispielen sind die Membranen vorzugsweise so mikrostrukturiert, dass die Kapillare als feine Kanäle eingeprägt sind, wenn die Membrane sich im noch verformbaren Zustand befindet im Fertigungsprozess.

In der in Figur 6 dargestellten Ausführung ist die Kanalführung durch mäanderförmig angeordnete, geprägte Kanäle 40 ausgebildet. Aus einem - im Ausführungsbeispiel als flexibler Beutel - ausgebildetes Wasserreservoir 49 wird mittels eines Einlasses 46 Wasser zugeführt.

Die Kanalquerschnitte können rechteckig, rund, oval oder eine x-beliebiger Form sein. Die Dimensionen der Kanalquerschnitte können je nach gewünschter Saugwirkung von 10 nm (entspricht Blattspaltöffnung eines Baumblattes) bis ca. 1 mm reichen. Der Querschnitt liegt im Bereich von ca. 10 bis 200 µm liegen. Im hier beschriebenen Ausführungsbeispiel werden die Dicken der Kanäle variiert, um so einen Gradient zu bekommen, analog dem menschlichen Gefässsystem, das heisst, dass die zuführenden Kanäle sind etwas dicker als die abführenden Kanäle. Die Kanaldicke beim Einlass 46 beträgt 100 µm und wird schrittweise - auf ca. 20 µm reduziert und endet in einem - im Ausführungsbeispiel als Entlüftungsmembrane ausgebildeten - Entlüftungspunkt 48. Zu Beginn einer Befüllung mit Wasser ist Luft in den kapillaren Hohlräumen des Kühlelementes enthalten, welche zunächst aus dem System verdrängt werden muss. Diese Luft wandert dann langsam - von der Wasserfront verdrängt - zu den Entlüftungslöchern, welche mit Mikroporösen Membranen verschlossen sind. Dadurch kann die Luft entweichen, aber das Wasser wird zurückgehalten.

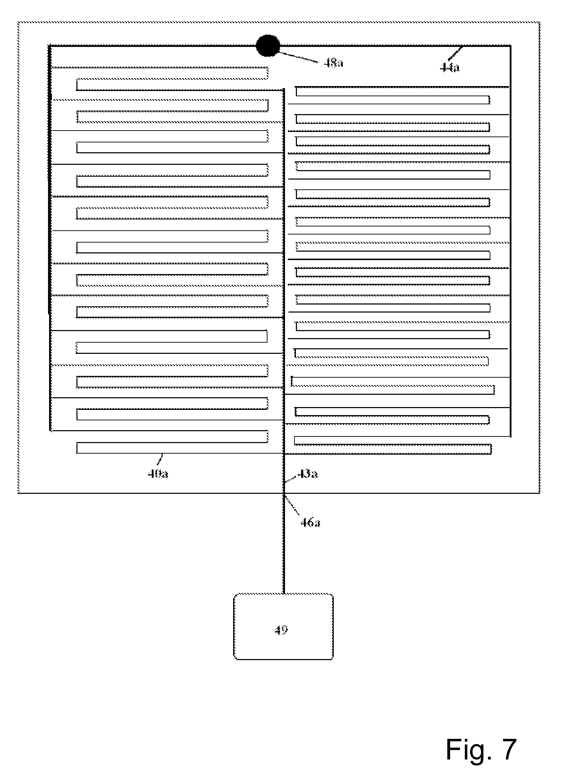

In einer ersten Variante der Kanalführung sind - wie in Figur 7 dargestellt - die Kanäle 40a als Kapillare funktionell zwischen einem - oder mehreren - Zuführkanal 43a und einem - oder mehrerer - Rückflusskanal 44a - analog des Kapillartransportes bei menschlichen Blutgefässen - angeordnet, wobei der Zuführkanal mit dem Einlass 46a und die Rückführung mit der Entlüftung - nämlich der Entlüftungsmembran 48a - verbunden ist.

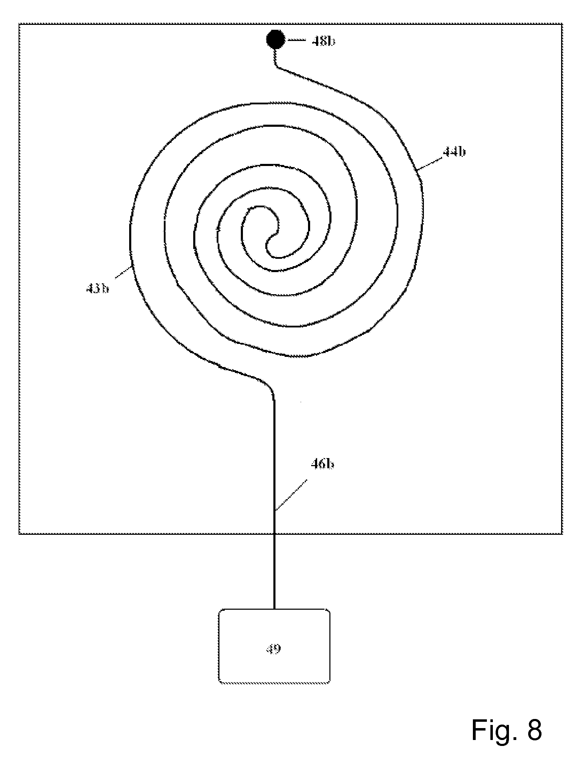

In einer zweiten Variante der Kanalführung sind Kanäle als Kapillare - wie in Figur 8 dargestellt -spiralförmig angeordnet. In dem in Figur 8 dargestellten Ausführungsbeispiel sind die Kanäle als zwei ineinander angeordneten Spiralen ausgebildet, wobei eine Spirale funktionell als Zuführkanal 43b und eine als Rückflusskanal 44b ausgebildet ist, wobei der Zuführkanal 43b wiederum mit dem Einlass 46b und die Rückführung mit der Entlüftung - nämlich der Entlüftungsmembran 48b - verbunden ist.

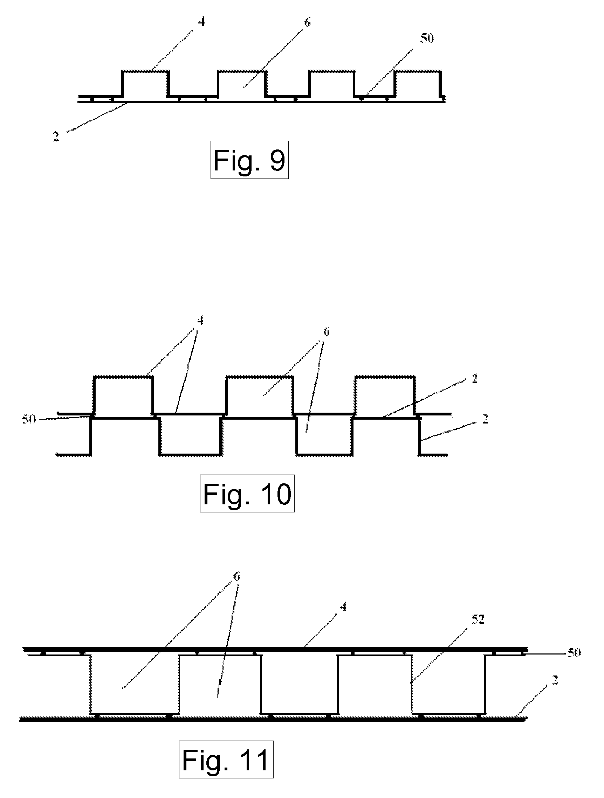

Beispiele für den Schichtenverbund der einzelnen strukturierten und unstrukturierten Membranen sind:
a) eine Membrane 4 wird mikrostrukturiert und die andere, normale Membrane 4 wird mit dieser mittels Hotmeltkleber 50 verbunden, wobei durch die Mikrostruktur die Verdunstungsschicht 6 ausgebildet wird (Figur 9);
b) zwei mikrostrukturierte Membranen 2 und 4 werden leicht zueinander versetzt und miteinander mittels Hotmeltkleber 50 verbunden (Figur 10);
c) die mittlere Membrane 52 wird mikrostrukturiert und auf beiden Seiten mit normalen Membranen2 und 4 mittels Hotmeltkleber 50 verbunden, sodass zwei unabhängige Wasserkreisläufe entstehen (Figur 11).

Es sollte betont werden, dass diese alternative Ausgestaltung des Kühlelementes einen erfinderischen Beitrag unabhängig vom Ventilationselement leistet, also auch mit einem anderen Ventilationskonzept oder ganz ohne ein solches ausgeführt werden kann, also ein flächiges Kühlelement, wobei das flächige Kühlelement ausgebildet ist mit zwei Aussenlagen sowie einem dazwischen angeordneten Verdunstungsbereich, wobei die Aussenlagen je aus einem wasserdichten und wasserdampfdurchlässigen Material gebildet sind und wobei Wasserzuführmittel vorhanden sind, um den Verdunstungsbereich mit flüssigem Wasser zu versorgen, wobei der Verdunstungsbereich durch in die zumindest eine der beiden Aussenlagen eingeprägte, kanalartige Strukturen, insbesondere durch Kapillaren ausgebildet ist. Die flächige Ausgestaltung der eingeprägten kanalartigen Struktur können als Spiralen oder mäanderförmige Strukturen ausgebildet sein und/oder die Dicken der kanalartigen Struktur können variieren, typischerweise so, dass die zuführenden Kanäle etwas dicker als die abführenden Kanäle sind.

In einer alternativen - hier nicht dargestellten- Ausführung wird statt einer Membrane ein feinmaschiges Gitter auf die mittlere Verteilschicht geklebt. Dabei hat sich der Vorteil gezeigt, dass die Entlüftung sehr gut funktioniert und eine mechanische Verletzung praktisch keinen Einfluss hat. Allerdings kann dann selbstverständlich kein kapillarer Saugeffekt mehr erzielt werden. Durch die luftdurchlässige Struktur des Maschengitters wird gewissermassen das Element dauernd belüftet. Weiterhin ergibt sich für die Befüllung, dass der Druck nicht zu hoch sein darf, da sonst, dass Wasser sofort raus durch das Maschengitter fliesst. Es ist also eine Befüllung mit sehr geringem Druck erforderlich.

## Patentansprüche

1. Kühlvorrichtung, umfassend ein flächiges Kühlelement (E) und ein flächiges Ventilationselement (VE), wobei das flächige Kühlelement (E) dreilagig ausgebildet ist mit zwei Aussenlagen (2, 4) sowie einem dazwischen angeordneten Verdunstungsbereich (6), wobei die Aussenlagen je aus einem wasserdichten und wasserdampfdurchlässigen Material gebildet sind und wobei Wasserzuführmittel (8) vorhanden sind, um den Verdunstungsbereich mit flüssigem Wasser zu versorgen, wobei das flächige Ventilationselement (V) eine Luft durchströmbare, Abstand haltende Ventilationsschicht aufweist, auf deren einen Seite zumindest bereichsweise eine zumindest weitgehend luftdichte Membrane (M) und auf deren anderen Seite eine aus einem wasserdampfdurchlässigen Material gebildete Schicht (30) des Ventilationselementes (V) ausgebildet ist, wobei das Kühlelement (E) und das Ventilationselement (V) an jeweils einer ihrer Flächen so aneinander angeordnet sind, dass eine der Aussenlagen (4) des Kühlelementes und die aus wasserdichtem und wasserdampfdurchlässigem Material gebildete Schicht (30) benachbart aneinander anliegen, und wobei zudem mindestens ein Aktivlüfter (L) vorhanden ist, mittels welchem das Ventilationselement von einer Lufteintrittzone (12) zu einer Luftaustrittzone (14) durchlüftbar ist, um aus dem Kühlelement (E) durch die Aussenlage (4) entwichenen und durch die aus wasserdichtem und wasserdampfdurchlässigem Material gebildete Schicht (30) in das Ventilationselement eingedrungenen Wasserdampf abzuführen.

2. Kühlvorrichtung nach Anspruch 1, **dadurch gekennzeichnet, dass** zwischen dem Kühlelement (E) und dem Ventilationselement (V) durch geeignete Mittel ein Abstand ausgebildet ist.

3. Kühlvorrichtung nach einem der Ansprüche 1 oder 2, **dadurch gekennzeichnet, dass** das Kühlelement (E) und das Ventilationselement (V) zumindest abschnittsweise, insbesondere in ihren Aussenbereichen, miteinander verbunden sind.

4. Kühlvorrichtung nach Anspruch 1, **dadurch gekennzeichnet, dass** das Ventilationselement (V) ein Abstandsgewirke (A), ein Abstandsgewebe und/oder Abstandshaltende lokale Elemente zum Ausbilden einer Ventilationsschicht enthält.

5. Kühlvorrichtung nach einem der Ansprüche 1 bis 4, **dadurch gekennzeichnet, dass** das Ventilationselement mit luftdichten seitlichen Abschlüssen versehen ist.

6. Kühlvorrichtung nach einem der Ansprüche 1 bis 5, **dadurch gekennzeichnet, dass** die Luftaustrittzone des Ventilationselementes (V) durch einen von der luftdichten Membrane freigelassenen Bereich des Ventilationselementes gebildet ist.

7. Kühlvorrichtung nach einem der Ansprüche 1 bis 6, **dadurch gekennzeichnet, dass** der Aktivlüfter in das Ventilationselement (V) integriert ist.

8. Kühlvorrichtung nach einem der Ansprüche 1 bis 7, **dadurch gekennzeichnet, dass** der Aktivlüfter ein batteriebetriebener Axial- oder Radiallüfter ist.

9. Kühlvorrichtung nach einem der Ansprüche 1 bis 8, **gekennzeichnet durch** eine Vorrichtung zur Steuerung der Wasserzuführmittel und/oder des Aktivlüfters.

10. Kühlvorrichtung nach einem der Ansprüche 1 bis 9, **dadurch gekennzeichnet, dass** die Dicke der dem Ventilationselement (V) zugewandten Aussenlage (4) des Kühlelementes (E) geringer ist als die Dicke der von dem Ventilationselement (V) abgewandten Aussenlage (2).

11. Kühlvorrichtung nach einem der Ansprüche 1 bis 10, **dadurch gekennzeichnet, dass** der Verdunstungsbereich (6) durch in die zumindest eine der beiden Aussenlagen (2,4) eingeprägte, kanalartige Strukturen, insbesondere durch Kapillaren ausgebildet ist.

12. Kühlvorrichtung nach Anspruch 11, **dadurch gekennzeichnet, dass** die flächige Ausgestaltung der eingeprägten kanalartigen Struktur als Spiralen oder mäanderförmige Strukturen ausgebildet ist.

13. Kühlvorrichtung nach Anspruch 11 oder 12, **dadurch gekennzeichnet, dass** die Dicken der kanalartigen Struktur variieren, wobei vorzugsweise die zuführenden Kanäle etwas dicker als die abführenden Kanäle sind.

14. Kühlendes Objekt, umfassend eine integrierte Kühlvorrichtung (K) nach einem der Ansprüche 1 bis 13.
